# DEMANDE DE BREVET EUROPEEN

(11) **EP 2 399 532 A1**
(43) Date de publication de la demande: **28.12.2011**
(21) Numéro de dépôt: 10167236.8
(22) Date de dépôt: 24.06.2010
(51) Int. Cl.: A61B 17/64

(54) **Machoire pour un fixateur externe et articulation utilisant de telles machoires**

(71) Demandeur: Bentele, Franz, 1202 Genève (CH)
(72) Inventeur: Bentele, Franz, 1202 Genève (CH)
(74) Mandataire: Leman Consulting S.A.

(57) **Abrégé**

La présente invention concerne une mâchoire pour le maintien de tiges de fixation et/ou de broches d'un fixateur externe. Cette mâchoire comporte deux mors formant un passage prévu pour recevoir la tige et/ou la broche. La mâchoire est formée d'un premier élément de mâchoire et d'un second élément de mâchoire, ces deux éléments de mâchoire étant mobiles l'un par rapport à l'autre le long d'un axe longitudinal. L'invention est caractérisée en ce que le premier élément de mâchoire comporte un logement ayant une ouverture s'étendant dans une direction opposée audit passage ainsi qu'une paroi, en ce que le second élément de mâchoire comporte un logement ayant une ouverture disposée en regard du passage ainsi qu'une paroi, la paroi du premier élément de mâchoire étant disposée dans ledit logement du second élément de mâchoire lorsque les deux éléments de mâchoire sont assemblés pour former la mâchoire, en ce que la paroi du second élément de mâchoire est disposée dans ledit logement du premier élément de mâchoire lorsque les deux éléments de mâchoire sont assemblés pour former la mâchoire, et en ce qu'un élément élastique est disposé entre lesdites parois du premier et du second élément de mâchoire.

La présente invention concerne également une articulation d'un fixateur externe utilisant au moins deux mâchoires telles que définies ci-dessus.

## Description

### DOMAINE TECHNIQUE

La présente invention concerne une mâchoire pour le maintien de tiges de fixation et/ou de broches d'un fixateur externe, cette mâchoire comportant deux mors formant un passage prévu pour recevoir la tige et/ou la broche, la mâchoire étant formée d'un premier élément de mâchoire et d'un second élément de mâchoire, ces deux éléments de mâchoire étant mobiles l'un par rapport à l'autre le long d'un axe longitudinal.

La présente invention concerne également une articulation d'un fixateur externe utilisant des mâchoires telles que définies ci-dessus.

### TECHNIQUE ANTERIEURE

Il existe actuellement différents types de mâchoires utilisés comme fixateur externe dans le domaine de l'orthopédie et/ou de la traumatologie. L'une de ces mâchoires est en particulier décrite dans le brevet européen N° EP 0 700 664. Ce document décrit une articulation destinée à recevoir des tiges et/ou des broches permettant de former une structure rigide pour le maintien d'un os lors de sa réparation après une fracture.

L'articulation décrite dans ce document est formée de deux mâchoires reliées par un axe et susceptibles de se déplacer le long de cet axe. Les deux mâchoires formant l'articulation sont séparées par un ressort de compression qui a pour effet d'écarter les mâchoires l'une de l'autre le long de l'axe. Ceci permet d'écarter les mors de la mâchoire et donc d'introduire facilement une tige ou une broche dans un passage des mâchoires, en utilisant l'élasticité du ressort.

Cette réalisation présente différents inconvénients. En premier lieu, la réalisation de la mâchoire est relativement complexe. En effet, le ressort est placé dans un logement qu'il est nécessaire de réaliser à cet usage, la forme du logement étant assez complexe. En second lieu, l'articulation selon l'invention nécessite impérativement la présence de deux mâchoires. En effet, étant donné que le ressort est disposé entre deux mâchoires, l'effet de poussée de l'une des mâchoires en direction de l'autre ne peut être obtenu que dans ce cas. Il en résulte qu'une mâchoire seule ne peut pas être utilisée. L'utilisation d'une seule mâchoire pourrait toutefois être intéressante lorsqu'il est nécessaire de relier deux tiges alignées par exemple. De même, il n'est pas possible d'utiliser plus de deux mâchoires, par exemple trois. Ceci pourrait être intéressant lorsqu'il est souhaitable de créer une structure particulièrement rigide par exemple.

La présente invention se propose donc de réaliser une mâchoire plus facile à produire et qui peut être utilisée seule, pour former une articulation comportant deux mâchoires, ou pour former une articulation comportant plus de deux mâchoires.

### EXPOSE DE L'INVENTION

Le but de l'invention est atteint par une mâchoire telle que définie en préambule et caractérisée en ce que le premier élément de mâchoire comporte un logement ayant une ouverture s'étendant dans une direction opposée audit passage ainsi qu'une paroi, en ce que le second élément de mâchoire comporte un logement ayant une ouverture disposée en regard du passage ainsi qu'une paroi, la paroi du premier élément de mâchoire étant disposée dans ledit logement du second élément de mâchoire lorsque les deux éléments de mâchoire sont assemblés pour former la mâchoire, en ce que la paroi du second élément de mâchoire est disposée dans ledit logement du premier élément de mâchoire lorsque les deux éléments de mâchoire sont assemblés pour former la mâchoire, et en ce qu'un élément élastique est disposé entre lesdites parois du premier et du second élément de mâchoire.

Le but de l'invention est également atteint par une articulation d'un fixateur externe caractérisée en ce qu'elle comporte au moins deux mâchoires telles que définies ci-dessus.

La mâchoire selon l'invention est formée de deux éléments de mâchoire qui peuvent se déplacer l'un par rapport à l'autre le long d'un axe longitudinal. En position libre, c'est-à-dire lorsqu'aucune force extérieure n'est appliquée, les deux éléments de mâchoire sont pressés l'un contre l'autre. Ceci permet de maintenir la tige ou la broche entre deux mors de la mâchoire. Ces deux éléments de mâchoire peuvent être écartés l'un de l'autre de manière à permettre l'introduction d'une tige ou d'une broche sans devoir dévisser une vis, mais simplement en poussant la tige ou la broche dans le passage de la mâchoire.

Etant donné que la partie élastique agit sur les constituants d'une seule mâchoire, la mâchoire selon l'invention peut être utilisée seule ou en combinaison avec d'autres mâchoires. Lorsque la tige ou la broche est introduite dans le passage adéquat, elle est maintenue par les mâchoires sans qu'il soit nécessaire qu'une personne la maintienne.

### DESCRIPTION SOMMAIRE DES DESSINS

La présente invention et ses avantages seront mieux compris en référence aux figures annexées et à la description détaillée d'un mode de réalisation particulier, dans lesquelles :
- la figure 1 est une vue de profil d'une mâchoire selon la présente invention;
- la figure 2 est une vue de profil d'une articulation de l'invention;
- la figure 3 et une vue de dessous d'un élément de la mâchoire de la figure 1;
- la figure 4 est une vue de dessus d'un autre élément de mâchoire de la figure 1;
- la figure 5 est une vue de profil de l'élément de mâchoire de la figure 3; et
- la figure 6 est une vue de profil de l'élément de mâchoire de la figure 4.

### MANIERE DE REALISER L'INVENTION

La mâchoire 10 selon l'invention est destinée au maintien de tiges 11 ou de broches 12 d'un fixateur externe. En référence aux figures, la mâchoire 10 comporte un premier élément de mâchoire 13 et un second élément de mâchoire 14 qui, lorsqu'ils sont assemblés, définissent un passage 15 destiné à recevoir une tige ou une broche. Ces éléments de mâchoire comportent un trou traversant 16 destiné à recevoir une vis 17 ou une tige filetée.

Le premier élément de mâchoire 13 comporte un logement 18 ayant une paroi 19 disposée en direction du milieu de la mâchoire, et une ouverture 20 s'étendant dans une direction opposée au passage 15. Dans le mode de réalisation illustré, la paroi 19 comporte une gorge 21 rectiligne dont la fonction est expliquée ci-dessous.

Le premier élément de mâchoire 13 peut également comporter une zone dentée 22 dont la fonction est également expliquée plus bas.

Le second élément de mâchoire 14 comporte également un logement 23 ayant une paroi 24 disposée en direction du milieu de la mâchoire, et une ouverture 25. L'ouverture 25 s'étend dans la direction du passage 15.

Les deux éléments de mâchoire 13, 14 ont chacun un mors 25, respectivement 26 à l'extrémité du passage 15, ces mors étant destinés à coopérer pour maintenir la tige 11 ou la broche 12.

Lorsque les deux éléments de mâchoire 13, 14 sont assemblés pour former la mâchoire 10, la paroi 19 du logement 18 du premier élément de mâchoire 13 est positionnée dans le logement 23 du second élément de mâchoire 14. De manière similaire, la paroi 24 du logement 23 du second élément de mâchoire 14 est placé dans le logement 18 du premier élément de mâchoire 13. Un élément élastique 27 est placé entre les deux parois 19, 24 des logements. Plus précisément, l'élément élastique 27 est introduit dans la gorge 21 du premier élément de mâchoire 13, de telle sorte que cet élément élastique ne risque pas de tomber. Il est à préciser que la gorge 21 est rectiligne. Elle est donc particulièrement facile à réaliser.

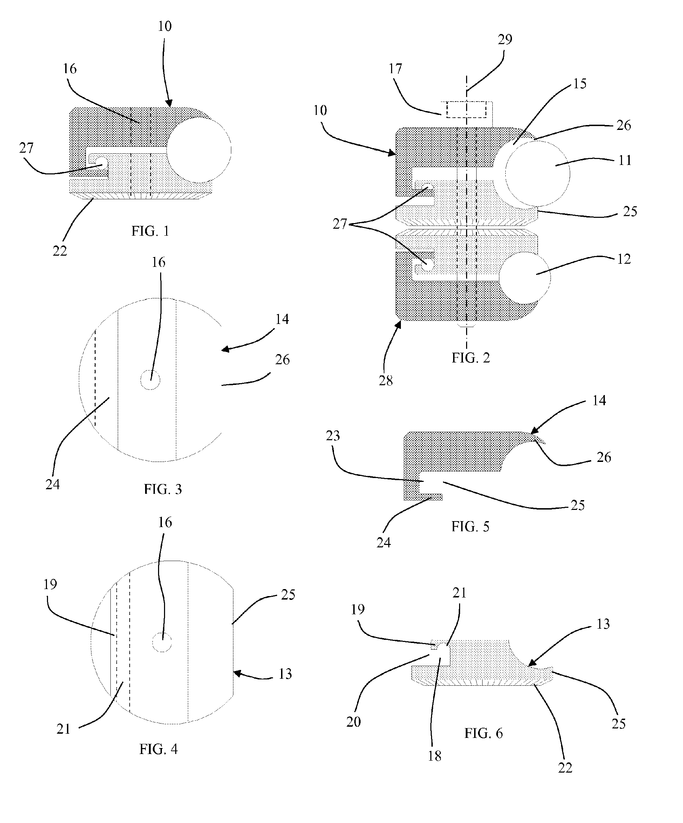

La figure 1 représente la mâchoire 10 en position de repos. Dans cette position, l'élément élastique 27 agit sur les parois 19, 24 des logements 18, 23 de façon à écarter ces parois l'une de l'autre sous l'effet de son élasticité. Ceci a pour effet d'approcher les deux éléments de mâchoire 13, 14 l'un de l'autre et de permettre le maintien d'une tige 11 entre les mors 25, 26 de la mâchoire. Il est à noter que le passage 15 défini par les mors couvre un arc de cercle légèrement plus grand qu'un demi cercle, ceci afin d'assurer un bon maintien de la tige ou de la broche. Il couvre toutefois un arc de cercle suffisamment petit pour qu'une pression de la tige contre les mors ait pour effet d'écarter les mors le long de la vis 17.

La figure 2 représente une articulation 28 formée de deux mâchoires 10 et d'une vis 17. La partie supérieure de cette figure représente la mâchoire 10 dans une position dans laquelle une force agit pour séparer les deux éléments de mâchoire. La force peut être produite par exemple lorsqu'un utilisateur introduit une tige ou une broche entre les mors 25, 26 de la mâchoire. Dans cette configuration, l'élément élastique 27 est comprimé entre les deux parois 19, 24 des logements. La dimension des logements 18, 23, l'épaisseur des parois 19, 24 et l'écartement des mors 25, 26 sont tels que lorsque l'élément élastique est comprimé, l'écartement des mors est supérieur ou égal au diamètre de la tige 11 ou de la broche 12 à introduire dans le passage 15. Les dimensions précises peuvent être variables, de même que les rapports entre ces dimensions. L'homme du métier pourra toutefois aisément déterminer ces dimensions pour que d'une part, la broche ou la tige soit maintenue sans nécessiter de force extérieure lorsque la mâchoire est laissée libre et que d'autre part, la broche ou la tige puisse être introduite dans le passage de la mâchoire en poussant simplement cette broche ou cette tige contre les mors dans une direction perpendiculaire à l'axe longitudinal.

Lorsque cette tige ou cette broche est introduite, l'élément élastique 27 agit sur les parois 19, 24 pour les écarter et ainsi resserrer les deux éléments de mâchoire 13, 14 et maintenir la tige ou la broche comme cela est expliqué en référence à la figure 1.

Les deux éléments de mâchoire peuvent s'écarter le long d'un axe longitudinal 29. Cet axe est matérialisé par la tige filetée ou la vis 17. La vis peut être utilisée avec un écrou (non représenté) pour bloquer le fixateur externe lorsque toutes les tiges et broches ont été mises en place dans la position souhaitée. Il est également possible de prévoir une zone filetée par exemple dans le premier élément de mâchoire 13 et un trou de diamètre supérieur au filetage dans le second élément de mâchoire 14. Dans ce cas, la vis peut être bloquée dans la zone filetée du premier élément de mâchoire sans utiliser un écrou.

Comme indiqué précédemment, le premier élément de mâchoire comporte une zone dentée 22. Celle-ci peut être utilisée avec une zone dentée d'une autre mâchoire. Ceci permet d'assurer un blocage des deux mâchoires l'une par rapport à l'autre dans une position angulaire choisie. Il est également possible de prévoir des zones dentées sur le deuxième élément de mâchoire, ce qui peut être utile lorsque plus de deux mâchoires sont utilisées sur un même axe.

La mâchoire selon la présente invention permet d'introduire et de retirer aisément et rapidement des tiges et des broches d'un fixateur externe. Ainsi, lorsque les broches sont positionnées dans le ou les os à maintenir, la structure du fixateur externe peut être rapidement mise en place, sans nécessiter l'aide de plusieurs personnes.

La mâchoire peut être utilisée en combinaison avec une autre mâchoire similaire pour former une articulation. Cette articulation peut être formée de deux mâchoires ou plus. La mâchoire peut également être utilisée seule. La mâchoire et l'articulation de l'invention offrent donc une grande souplesse et une efficacité accrue par rapport aux dispositifs connus.

## Revendications

1. Mâchoire pour le maintien de tiges de fixation et/ou de broches d'un fixateur externe, cette mâchoire comportant deux mors formant un passage prévu pour recevoir la tige et/ou la broche, la mâchoire étant formée d'un premier élément de mâchoire et d'un second élément de mâchoire, ces deux éléments de mâchoire étant mobiles l'un par rapport à l'autre le long d'un axe longitudinal, **caractérisée en ce que** le premier élément de mâchoire (13) comporte un logement (18) ayant une ouverture (20) s'étendant dans une direction opposée audit passage (15) ainsi qu'une paroi (19), **en ce que** le second élément de mâchoire (14) comporte un logement (23) ayant une ouverture (25) disposée en regard du passage (15) ainsi qu'une paroi (24), la paroi (19) du premier élément de mâchoire (13) étant disposée dans ledit logement (23) du second élément de mâchoire (14) lorsque les deux éléments de mâchoire sont assemblés pour former la mâchoire (10), **en ce que** la paroi (24) du second élément de mâchoire (14) est disposée dans ledit logement (18) du premier élément de mâchoire (13) lorsque les deux éléments de mâchoire sont assemblés pour former la mâchoire (10), et **en ce qu'**un élément élastique (27) est disposé entre lesdites parois (19, 24) du premier et du second élément de mâchoire.

2. Mâchoire selon la revendication 1, **caractérisée en ce que** la hauteur des logements (18, 23) dans le sens de l'axe longitudinal (29), l'épaisseur des parois (19, 24) et la dimension de l'élément élastique (27) sont tels que :
- la contrainte provoquée par l'élément élastique (27) approche les éléments de mâchoire (13, 14) l'un de l'autre de telle façon que ladite tige (11) et/ou ladite broche (12) soit maintenues entre les deux mors (25, 26),
- les deux éléments de mâchoire (13, 14) peuvent être écartés l'un de l'autre d'une distance permettant l'insertion d'une tige (11) et/ou d'une broche (12) dans le passage (15) de la mâchoire (10) dans une direction perpendiculaire à un axe de la tige et/ou de la broche.

3. Mâchoire selon la revendication 1, **caractérisée en ce que** la paroi (19) dudit premier élément de mâchoire (13) comporte une gorge (21) perpendiculaire audit axe longitudinal (29) et agencée pour recevoir ledit élément élastique (27).

4. Mâchoire selon l'une quelconque des revendications précédentes, **caractérisée en ce que** ladite paroi (24) du logement (23) du second élément de mâchoire (14) comporte une gorge perpendiculaire audit axe longitudinal (29) et agencée pour recevoir ledit élément élastique (27).

5. Mâchoire selon la revendication 1, **caractérisée en ce que** l'élément élastique (27) est un joint en élastomère.

6. Mâchoire selon la revendication 1, **caractérisée en ce qu'**au moins l'un desdits éléments de mâchoire (13) comporte une zone dentée (22).

7. Mâchoire selon la revendication 1, **caractérisée en ce qu'**elle comporte un trou traversant (16) colinéaire avec ledit axe longitudinal (29).

8. Mâchoire selon la revendication 1, **caractérisée en ce que** ledit trou traversant (16) comporte une zone filetée dans l'un desdits éléments de mâchoire.

9. Articulation d'un fixateur externe **caractérisé en ce qu'**il comporte au moins deux mâchoires (10) telles que définies dans l'une quelconque des revendications précédentes.

10. Articulation selon la revendication 9, **caractérisé en ce que** chaque mâchoire (10) comporte une zone dentée (22), les zones dentées de deux mâchoires adjacentes étant disposées en regard l'une de l'autre.

11. Articulation selon la revendication 9 ou 10, **caractérisé en ce qu'**il comporte des moyens de blocage d'une mâchoire par rapport à l'autre.

12. Articulation selon la revendication 11, **caractérisé en ce que** les moyens de blocage comportent une vis (17) passant par un trou (16) traversant lesdites mâchoires.
